# EUROPÄISCHE PATENTSCHRIFT

(11) **EP 0 668 101 B1**
(45) Veröffentlichungstag und Bekanntmachung des Hinweises auf die Patenterteilung: **07.06.2000**
(21) Anmeldenummer: 95101215.2
(22) Anmeldetag: 30.01.1995
(51) Int. Cl.: B01J 23/64, C07C 209/72

(54) **Ruthenium-Katalysatoren, deren Herstellung und ein Verfahren zur Herstellung von cycloaliphatischen Polyaminen unter Verwendung dieser Katalysatoren**
Ruthenium catalysts, their preparation and their use in the preparation of cycloaliphatic polyamines
Catalyseurs au ruthénium, leur préparation et leur utilisation dans la préparation de polyamines cycloaliphatiques

(30) Priorität: 10.02.1994 DE 4404220
(43) Veröffentlichungstag der Anmeldung: 23.08.1995
(73) Patentinhaber: BAYER AG, 51368 Leverkusen (DE)
(72) Erfinder: Kiel, Wolfgang, Dr., D-51519 Odenthal (DE); Zirngiebl, Eberhard, Dr., D-51061 Köln (DE); Jentsch, Joerg Dietrich, Dr., D-45468 Mülheim an der Ruhr (DE)

(56) Entgegenhaltungen:
- EP-A- 0 001 425
- EP-A- 0 351 661
- EP-A- 0 476 359
- EP-A- 0 503 347
- US-A- 3 983 072

## Beschreibung

Die vorliegende Erfindung betrifft neuartige Ruthenium-Katalysatoren, Verfahren zu deren Herstellung und ein Verfahren zur Herstellung von cycloaliphatischen Polyaminen durch katalytische Hydrierung der entsprechenden aromatischen Amine unter Verwendung der neuartigen Ruthenium-Katalysatoren.

Es ist bekannt, daß man aromatische Polyamine in Gegenwart von Ruthenium-Katalysatoren hydrieren und so die entsprechenden cycloaliphatischen Polyamine herstellen kann. Die US-PS 4 849 544 beschreibt Ruthenium-Trägerkatalysatoren, die zusätzlich Alkalihydroxide, Alkalialkoxide oder Alkaliamid-Salze von m-Phenylendiamin enthalten und deren Verwendung zur Herstellung von 1,3-Diaminocyclohexanen aus m-Phenylendiaminen. Nachteilig hierbei sind die lange Reaktionszeit von 17 bis 24 Stunden und die teilweise niedrigen Ausbeuten, mit denen die 1,3-Diaminocyclohexane erhalten werden (siehe die Beispiele dieser US-PS).

Aus EP-A 0 476 359 und EP-A-0 351 661 ist ein Ruthenium-Katalysator bekannt, der 0,05 bis 5 Gew.-% Ruthenium auf einem Al₂O₃-Träger enthält, welcher mit Verbindungen von seltenen Erdmetallen, wie z.B. Cer, und Mangan behandelt wurde. Bei der Hydrierung aromatischer Amine weist dieser Katalysator allerdings teilweise keine sehr guten Selektivitäten und Ausbeuten auf.

Gute Ergebnisse erhält man, wenn man zur Herstellung cycloaliphatischer Polyamine aus den entsprechenden aromatischen Aminen Katalysatoren einsetzt, die Ruthenium und Hydroxide und/oder Oxidhydrate des Chroms und Mangans und/ oder deren Dehydratisierungsprodukte enthalten (siehe DE-OS 2 502 893). Auch aus EP-A-0 001 425 ist ein Ruthenium-Trägerkatalysator zur Hydrierung aromatischer Amine bekannt, wobei Hydroxide und/oder Oxide des Chroms und Mangans und/oder deren Dehydratisierungsprodukte als Trägermaterial fungieren und als Katalysator im Rahmen seiner Herstellung abschließend zwingend mit anorganischen und/oder organischen Alkaliverbindungen behandelt wird. Nachteilig bei den vorgenanten Katalysatoren ist jedoch, daß bei der Katalysatorherstellung Niederschläge anfallen, die nur sehr schwer filtrierbar sind. Außerdem müssen chromhaltige Abwässer entsorgt werden, was erheblichen Aufwand und Kosten bedeutet.

Cycloaliphatische Polyamine sind wichtige Ausgangsprodukte zur Herstellung von Polykondensations-Kunststoffen und Lacken, beispielsweise auf der Basis von Polyharnstoffen, Polyamiden und Polyurethanen. Sie werden auch als Härter in Epoxidharzen verwendet. Auf dem Lackgebiet haben cycloaliphatische Polyamine besondere Bedeutung erlangt, da sie von heller Farbe sind und den daraus hergestellten Lacken hohe Vergilbungsbeständigkeit und hohe Resistenz gegen agressive Chemikalien verleihen.

Es wurden nun Ruthenium-Katalysatoren gefunden, die im vorliegenden Anspruch 1 beschrieben werden.

Der Ruthenium-Gehalt erfindungsgemäßer Katalysatoren kann, berechnet als elementares Ruthenium, beispielsweise 0,1 bis 20 Gew.-% betragen. Vorzugsweise liegt dieser Gehalt im Bereich 0,5 bis 5 Gew.-%.

Das Gewichtsverhältnis Cer zu Mangan, ebenfalls berechnet als Elemente, kann beispielsweise 0 bis 5:1 betragen. Vorzugsweise liegt dieses Verhältnis im Bereich 0,1 bis 3:1, besonders bevorzugt im Bereich 0,8 bis 2:1.

Erfindungsgemäße Katalysatoren müssen außer Ruthenium in metallischer oder gebundener Form und Carbonaten, Hydroxiden und/oder Oxidhydraten des Cers und Mangans und/oder deren Dehydratisierungsprodukten keine weiteren Bestandteile enthalten. Sie können jedoch auch bei der Katalysatorherstellung übliche Zusatzstoffe und/oder Hilfsmittel enthalten, beispielsweise Wasser, Tablettierungshilfsmittel, Bindemittel, Porosierungsmittel und/oder Füllstoffe. Sie dürfen jedoch kein Chrom enthalten.

Erfindungsgemäße Katalysatoren kann man beispielsweise herstellen, indem man lösliche Verbindungen des Rutheniums, Cers und Mangans durch Behandlung mit alkalisch reagierenden Verbindungen in unlösliche Verbindungen überführt und diese gegebenenfalls nachbehandelt. Beispielsweise kann man die essentiellen Katalysatorkomponenten gemeinsam in unlösliche Verbindungen überführen. Man kann auch zunächst nur lösliche Cer-und Manganverbindungen oder nur Manganverbindungen in unlösliche Verbindungen überführen, diese gegebenenfalls nachbehandeln, z.B. abfiltrieren und trocknen, und dann eine lösliche oder unlösliche Rutheniumverbindung darauf aufbringen und gegebenenfalls nochmals nachbehandeln. Als letztgenannte Nachbehandlung kann beispielsweise eine Behandlung mit Reduktionsmitteln durchgeführt werden. Hierfür geeignete Reduktionsmittel sind z.B. Hydrazinhydrat, Ameisensäure, Formiate und Ascorbinsäure.

Als lösliche Verbindungen von Ruthenium, Cer und Mangan kommen insbesondere Ruthenium(III)-nitrat, Ruthenium(III)-chlorid, Cer(III)-nitrat und Mangan(II)-nitrat in Frage, die gegebenenfalls Hydratwasser enthalten können. Die Überführung in unlösliche Rutheniumverbindungen und Carbonate, Hydroxide oder Oxidhydrate des Cers und Mangans erfolgt durch Zusätze von alkalisch reagierenden Verbindungen. Beispielsweise kommen hier Alkalicarbonate und Alkalihydroxide in Frage.

Gegebenenfalls kann eine Nachbehandlung durch Filtration, Freiwaschen von löslichen Bestandteilen, Wärmebehandlung, Formgebung und/oder die vollständige oder teilweise Reduktion des Rutheniums zum Metall erfolgen.

Das Freiwaschen von löslichen Bestandteilen kann beispielsweise so durchgeführt werden, bis im Waschwasser keine Anionen der eingesetzten löslichen Verbindungen mehr nachweisbar sind. Die Wärmebehandlung kann z.B. eine Trocknung bei Temperaturen bis zu beispielsweise 120°C, gegebenenfalls bei vermindertem Druck, sein. Nach einer solchen Trocknung kann gegebenenfalls eine Kalzinierung bei Temperaturen von beispielsweise 200 bis 450°C, vorzugsweise 250 bis 350°C, erfolgen.

Nach Trocknung und gegebenenfalls Kalzinierung, kann man erfindungsgemäße Katalysatoren gegebenenfalls mahlen, homogenisieren und, z.B. zusammen mit üblichen Hilfsmitteln, zu Formlingen, z.B. Tabletten, verformen. Es kann vorteilhaft sein, erfindungsgemäße Katalysatoren vor ihrer ersten Verwendung zu reduzieren, beispielsweise durch eine Behandlung mit Wasserstoff bei 20 bis 200°C und Drucken von 0,1 bis 350 bar.

Für die Herstellung von pulverförmigen erfindungsgemäßen Katalysatoren kann es vorteilhaft sein, zunächst gemeinsam die Carbonate, Hydroxide und/oder Oxidhydrate des Cers und Mangans und/oder deren Dehydratisierungsprodukte herzustellen und darauf die Abscheidung des Rutheniums oder von Rutheniumverbindungen vorzunehmen. Bei der Herstellung von erfindungsgemäßen stückigen Katalysatoren kann man beispielsweise so vorgehen, daß man ein gemeinsam hergestelltes dehydratisiertes Cer- und Manganhydroxid und/oder entsprechende Oxidhydrate nach der Verformung, beispielsweise Tablettierung, nacheinander mit einer Rutheniumsalzlösung und einer Alkalicarbonat- oder -hydroxidlösung in beliebiger Reihenfolge tränkt, jeweils nach der Tränkung eine Zwischentrocknung vornimmt und anschließend mit Wasser auswäscht. Man kann auch die Rutheniumsalzlösung und Alkalicarbonat- oder -hydroxidlösung nacheinander in einer beheizten Dragiertrommel auf geformtes und dehydratisiertes Cer- und Manganhydroxid und/oder entsprechende Oxidhydrate aufsprühen und diese dann waschen. Die Anzahl der Tränk- oder Dragierschritte richtet sich nach der aufzubringenden Menge von Ruthenium.

Die vorliegende Erfindung betrifft weiterhin ein Verfahren zur Herstellung von cycloaliphatischen Polyaminen durch katalytische Hydrierung von entsprechenden aromatischen Aminen in Gegenwart eines Ruthenium-Katalysators, das dadurch gekennzeichnet ist, daß man einen Katalysator verwendet, der im vorliegenden Anspruch 1 beschrieben wird.

Weitere Details der einzusetzenden Katalysatoren und Möglichkeiten zu deren Herstellung sind weiter oben beschrieben.

In das erfindungsgemäße Verfahren können z.B. aromatische Amine der Formel (I) eingesetzt werden 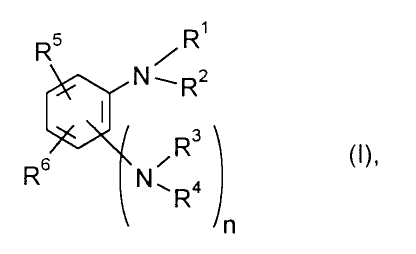 in der
R¹, R², R³ und R⁴ gleich oder verschieden sind und jeweils für Wasserstoff oder C₁-C₁₀-Alkyl stehen,
R⁵ und R⁶ gleich oder verschieden sind und jeweils für Wasserstoff, Hydroxyl, C₁-C₁₀-Alkyl, C₅-C₁₀-Cycloalkyl oder C₆-C₁₄-Aryl stehen, wobei diese Alkyl-, Cycloalkyl- und Arylreste gegebenenfalls mit Hydroxy- und/oder NH₂-Gruppen substituiert sein können und
n für 1 oder 2 steht.

Bevorzugte Alkylreste sind Methyl, Ethyl, Propyl, Isopropyl, Butyl, Isobutyl, tert.-Butyl, Pentyl und Hexyl.

Bevorzugte Cycloalkyle sind Cyclopentyl und Cyclohexyl.

Bevorzugte Arylreste sind Phenyl und Naphthyl.

Besonders bevorzugte Verbindungen der Formel (I) sind:
2,4-, 2,6-, 2,3- und 2,5-Diamino-methylbenzol und Gemische, die diese Verbindungen enthalten,
2,4-, 2,6-, 2,5- und 2,3-Diamino-isopropylbenzol und Gemische, die diese Verbindungen enthalten,
2,4- und 2,6-Diamino-3,5-diethyl-methylbenzol und Gemische, die diese Verbindungen enthalten,
2,4,6-Triamino-methylbenzol, 2,4-Diamino-isobutylbenzol und o-, m- und p-Phenylendiamin sowie 2,4-Diaminophenol.

In das erfindungsgemäße Verfahren können z.B. auch aromatische Amine der Formel (II) eingesetzt werden 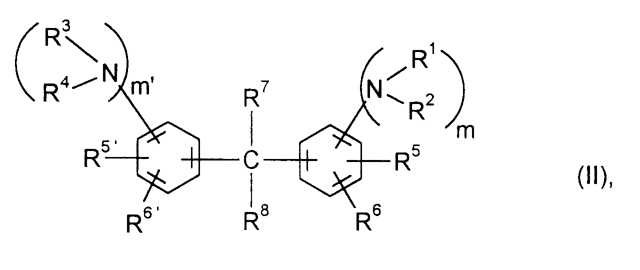 in der
R¹ bis R⁶ die bei Formel (I) angegebene allgemeine und bevorzugte Bedeutung haben,
R^{5'} und R^{6'} dem Umfang nach die bei Formel (I) für R⁵ und R⁶ angegebene allgemeine und bevorzugte Bedeutung haben, jedoch von R⁵ und R⁶ verschieden sein können,
R⁷ und R⁸ gleich oder verschieden sind und jeweils für Wasserstoff oder C₁-C₁₀-Alkyl stehen und
m und m' gleich oder verschieden sind und jeweils für Null, 1 oder 2 stehen, wobei
m + m' mindestens 2 ist.

R⁷ und R⁸ sind vorzugsweise gleich und stehen vorzugsweise für Methyl, Ethyl oder Isopropyl.

Besonders bevorzugte Verbindungen der Formel (II) sind 3,4,4'-Triamino-3,3'-dimethyl-diphenylmethan, 2,4,4'-Triamino-3,3'-diisopropyldiphenylmethan, 2,4,4'-Triamino-3-isopropyldiphenylmethan, 2,4,4'-Triamino-3-methyl-diphenylmethan und 2,2',4,4'-Tetraamino-3,3'-dimethyldiphenylmethan.

In das erfindungsgemäße Verfahren können z.B. auch aromatische Amine der Formel (III) eingesetzt werden 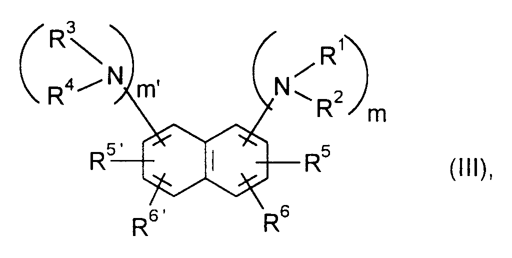 in der
R¹ bis R⁶ die bei Formel (I) angegebene allgemeine und bevorzugte Bedeutung haben,
R^{5'} und R^{6'} die bei Formel (II) angegebene allgemeine und bevorzugte Bedeutung haben und
m und m' die bei Formel (II) angegebene Bedeutung haben, wobei
m + m' mindestens 2 ist.

Das erfindungsgemäße Verfahren wird im allgemeinen bei erhöhter Temperatur und bei erhöhtem Druck durchgeführt, beispielsweise bei 100 bis 360°C und 50 bis 400 bar, vorzugsweise 180 bis 300°C und 150 bis 360 bar.

Im allgemeinen kann man das erfindungsgemäße Verfahren ohne den Zusatz von Lösungsmitteln durchführen. Insbesondere, wenn über der vorgesehenen Reaktionstemperatur schmelzende aromatische Amine eingesetzt werden sollen, kann man auch Lösungsmittel zusetzen, beispielsweise Cyclohexan, Methylcyclohexan und/oder tert.-Butanol.

Die Menge des Katalysators kann beispielsweise so bemessen werden, daß im Katalysator pro kg eingesetztes aromatisches Amin 0,05 bis 5 g Ruthenium (gerechnet als Metall) vorliegen. Vorzugsweise beträgt diese Menge 0,1 bis 2 g, insbesondere 0,2 bis 1 g.

Man kann das erfindungsgemäße Verfahren kontinuierlich oder diskontinuierlich durchführen. Für die diskontinuierliche Arbeitsweise kommen beispielsweise Rührautoklaven oder Schlaufenreaktoren in Frage, in denen man den Katalysator, vorzugsweise in Pulverform, einbringt und vorzugsweise in der Sumpfphase arbeitet.

Vorzugsweise wird das erfindungsgemäße Verfahren kontinuierlich durchgeführt, beispielsweise mit pulverförmigen Katalysator nach dem Blasensäulenprinzip, indem man flüssiges oder gelöstes aromatisches Amin, in dem der Katalysator suspendiert ist, zusammen mit Wasserstoff im Gleichstrom durch eine Reaktionskaskade leitet. Man kann beispielsweise auch mit stückigem Katalysator in der Weise arbeiten, daß man flüssiges oder gelöstes aromatisches Amin über einen in einem Reaktionsrohr fest angeordneten Katalysator rieseln läßt, während Wasserstoff im Gleich- oder Gegenstrom durch das Reaktionrohr geleitet wird.

Insbesondere bei kontinuierlicher Fahrweise ist es vorteilhaft, Wasserstoff im Überschuß einzusetzen, und nicht umgesetzten Wasserstoff im Kreis zu führen.

Es sind auch noch andere Ausführungsformen für das erfindungsgemäße Verfahren möglich.

Auf die erfindungsgemäße Weise erhält man aus den eingesetzten aromatischen Aminen die entsprechenden cycloaliphatischen Polyamine. Beispielsweise erhält man aus aromatischen Aminen der Formel (I) cycloaliphatische Polyamine der Formel (Ia) 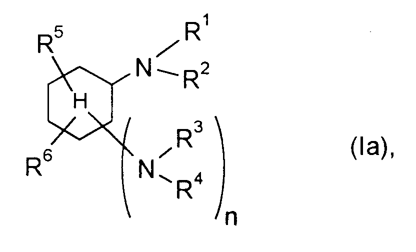 in der die verwendeten Symbole die bei Formel (I) angegebene Bedeutung haben.

Entsprechend erhält man aus aromatischen Aminen der Formel (II) cycloaliphatische Polyamine der Formel (IIa) 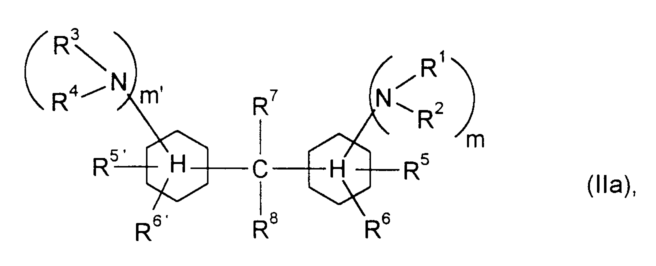 in der die verwendeten Symbole die bei Formel (II) angegebene Bedeutung haben und aus aromatischen Aminen der Formel (III) cycloaliphatische Polyamine der Formel (IIIa) 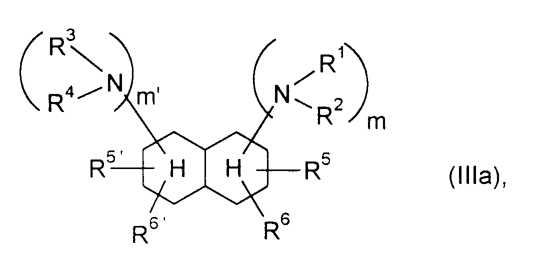 in der die verwendeten Symbole die bei Formel (III) angegebene Bedeutung haben.

Gegebenenfalls kann man das nach Durchführung des erfindungsgemäßen Verfahrens erhaltene cycloaliphatische Polyamin enthaltende Reaktionsgemisch noch weiter reinigen, z.B. durch Destillation und/oder Kristallisation.

Das erfindungsgemäße Verfahren hat eine Reihe von überraschenden Vorteilen: Es gestattet die Herstellung cycloaliphatischer Polyamine mit hohen Selektivitäten und mit kurzen Reaktionszeiten bzw. hoher Katalysatorbelastung. Die Katalysatorherstellung ist einfach, da nur gut filtrierbare Niederschläge und keine besonders zu entsorgenden Abwässer anfallen. Schließlich verliert der Katalysator auch bei längeren Laufzeiten nicht seine Selektivität und nicht die mit ihm erreichbare Hydriergeschwindigkeit. Häufig nimmt die Katalysatoraktivität bei längerer Laufzeit sogar zu.

### Beispiele

### Beispiel 1

7 l demineralisiertes Wasser wurde vorgelegt und 1074 g Ce(NO₃)₃ x H₂O, 1583 g Mn(NO₃)₂ x 4 H₂O und 146 g Ru(NO₃)₃ zugegeben. Die Feststoffe wurden unter Rühren aufgelöst und die Lösung auf 85°C erwärmt. Dann wurden bei dieser Temperatur 1201 g Na₂CO₃, gelöst in 5 l demineralisiertem Wasser zudosiert und schließlich 115,5 g Hydrazinhydrat (23,6 Gew.-%ig) gelöst in 4,5 l demineralisiertem Wasser. Die entstandene Suspension wurde 1,5 Stunden nachgerührt. Danach wurde abgekühlt und der ausgefallene Niederschlag filtriert. Der Filterkuchen wurde mit demineralisiertem Wasser Nitrat-frei gewaschen, bei 100 bis 110°C getrocknet und danach gemahlen. Abschließend wurde das entstandene Pulver 4 Stunden bei 300°C kalziniert.

### Beispiel 2

200 g eines Gemisches aus 65 Gew.-% 2,4- und 35 Gew.-% 2,6-Diaminotoluol wurden in einem 0,7 l Autoklaven bei 220 bis 240°C und bei 150 bis 180 bar in Gegenwart von 6,4 g des gemäß Beispiels 1 erhaltenen Katalysators bis zur Beendigung der Wasserstoffaufnahme hydriert. Die Reaktion war nach 2,5 Stunden beendet. Das Reaktionsgemisch enthielt 94 Gew.-% 2,4- und 2,6-Diaminomethylcyclohexan (Gemisch 65:35) und 5 Gew.-% Methylcyclohexylamin.

Die Filtrierzeit bei der Herstellung des Katalysators betrug 45 sec.

### Beispiel 3

Beispiel 2 wurde mehrmals wiederholt, wobei stets der Katalysator eingesetzt wurde, der bereits im vorangegangenen Einsatz zugegen war. Die Hydrierzeit und die Zusammensetzung des Reaktionsgemisches war wie aus der folgenden Tabelle ersichtlich ist:

| Bsp. Nr. | Katalysator aus Bsp.Nr. | Hydrierzeit [min] | im Reaktionsgemisch [Gew.-%] | |
|---|---|---|---|---|
| | | | Diaminomethyl-cyclohexan | Methylcyclohexylamin |
| 3a | 2 | 173 | 91,4 | 6,2 |
| 3b | 3a | 118 | 93,3 | 6,2 |
| 3c | 3b | 115 | 94,9 | 4,5 |

### Beispiel 4 (Vergleichsbeispiel)

Es wurde verfahren wie in Beispiel 2, jedoch gelangte ein gemäß Beispiel 1 der DE-OS 2 502 893 hergestellter Ru/Mn/Cr-Katalysator zum Einsatz. Die Reaktion war nach 293 Minuten beendet, das hydrierte Gemisch enthielt 93 Gew.-% Diaminomethylcyclohexan und 6 Gew.-% Methylcyclohexylamin.

Die Filtrierzeit bei der Herstellung des Katalysators betrug 5,5 min.

### Beispiel 5

100 g des auch in Beispiel 2 eingesetzten Toluylendiamin-Gemisches, 100 g Cyclohexan und 3,2 g des gemäß Beispiel 1 erhaltenen Katalysators wurden in einem Autoklaven bei 220°C und 180 bar mit Wasserstoff hydriert. Die Hydrierung war nach 320 min beendet. Danach enthielt das Reaktionsgemisch 92 Gew.-% Diaminomethylcyclohexan.

### Beispiel 6

200 g 2,4-Diaminocumol wurden in einem 0,7 l Autoklaven bei 230 bis 270°C und 150 bis 180 bar mit 6,4 g Katalysator erhalten gemäß Beispiel 1 bis zur Beendigung der Wasserstoffaufnahme hydriert. Die Reaktion war nach 60 Minuten beendet. Das hydrierte Gemisch enthielt danach 90,7 Gew.-% 2,4-Diaminoisopropylcyclohexan und 8,3 Gew.-% Isopropylcyclohexylamin.

### Beispiel 7

200 g 2,4-Diethyldiaminotoluol wurden in einem 0,7 l Autoklaven bei 240 bis 280°C und 150 bis 180 bar mit 6,4 g des gemäß Beispiel 1 erhaltenen Katalysators bis zur Beendigung der Wasserstoffaufnahme hydriert. Die Reaktion war nach 120 Minuten beendet. Das danach vorliegende hydrierte Gemisch enthielt 95,3 Gew.-% 2,4-Diethylmethyldiaminocyclohexan.

### Beispiel 8

200 g 2,4,6-Triisopropyl-1,3-diaminobenzol wurden in einem 0,7 l Autoklaven bei 240 bis 280°C und 150 bis 180 bar mit 6,4 g des Katalysators erhalten gemäß Beispiel 1 bis zur Beendigung der Wasserstoffaufnahme hydriert. Die Reaktion war nach 180 Minuten beendet. Das danach vorliegende hydrierte Gemisch enthielt 85,8 Gew.-% 2,4,6-Triisopropyl-1,3-diaminocyclohexan und 8,8 Gew.-% Triisopropylcyclohexylamin.

### Beispiel 9

Es wurde verfahren wie in Beispiel 2 beschrieben, jedoch wurde ein Katalysator eingesetzt, der wie in Beispiel 1 beschrieben hergestellt worden war, jedoch unter Weglassung des Cernitrats. Die Reaktion war nach 3 Stunden beendet und das Reaktionsgemisch enthielt 90,6 Gew.-% Diaminomethylcyclohexan und 5,6 Gew.-% Methylcyclohexylamin.

## Patentansprüche

1. Katalysatoren, dadurch gekennzeichnet, daß sie aus Ruthenium in metallischer oder gebundener Form und Carbonaten, Hydroxiden und/oder Oxidhydraten des Cers und Mangans und/oder deren Dehydratisierungsprodukten bestehen.

2. Katalysatoren nach Anspruch 1, dadurch gekennzeichnet, daß der Ruthenium-Gehalt 0,1 bis 20 Gew.-%, bezogen auf das Gesamtgewicht des Katalysators, beträgt (berechnet als elementares Ruthenium) und das Gewichtsverhältnis von Cer zu Mangan (berechnet als Elemente) 0 bis 5:1 beträgt.

3. Verfahren zur Herstellung von Ruthenium-Katalysatoren gemäß Anspruch 1, dadurch gekennzeichnet, daß man lösliche Verbindungen des Rutheniums, Cers und Mangans aber nicht des Chroms durch Behandlung mit alkalisch reagierenden Verbindungen in unlösliche Verbindungen überführt und diese gegebenenfalls nachbehandelt, mit der Maßgabe, daß bei dieser Herstellung der Ruthenium-Katalysatoren gemäß Anspruch 1 kein Al₂O₃-Träger anwesend ist.

4. Verfahren nach Anspruch 3, dadurch gekennzeichnet, daß man Ruthenium nachträglich auf die gegebenenfalls nachbehandelten unlöslichen Verbindungen des Cers und Mangans oder des Mangans aufbringt.

5. Verfahren nach Anspruch 3, dadurch gekennzeichnet, daß die Nachbehandlung durch Filtration, Freiwaschen von löslichen Bestandteilen, Wärmebehandlung, Formgebung und/oder Reduktion des Rutheniums erfolgt.

6. Verfahren nach Anspruch 5, dadurch gekennzeichnet, daß man eine Wärmebehandlung durchführt, bei der man bei Temperaturen bis zu 120°C trocknet und dann bei Temperaturen von 200 bis 450°C kalziniert.

7. Verfahren zur Herstellung von cycloaliphatischen Polyaminen durch katalytische Hydrierung von entsprechenden aromatischen Aminen in Gegenwart eines Katalysators, dadurch gekennzeichnet, daß man einen Katalysator verwendet, der aus Ruthenium in metallischer oder gebundener Form und Carbonaten, Hydroxiden und/oder Oxidhydraten des Cers und Mangans und/oder deren Dehydratisierungsprodukten besteht.

8. Verfahren nach Anspruch 7, dadurch gekennzeichnet, daß man aromatische Amine der Formel (I) einsetzt 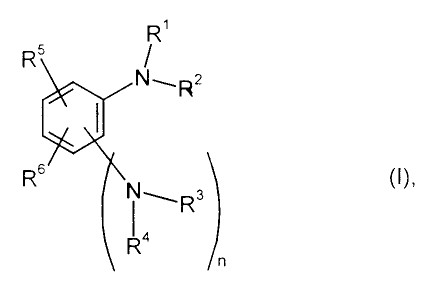 in der
R¹, R², R³ und R⁴ gleich oder verschieden sind und jeweils für Wasserstoff oder C₁-C₁₀-Alkyl stehen,
R⁵ und R⁶ gleich oder verschieden sind und jeweils für Wasserstoff, Hydroxyl, C₁-C₁₀-Alkyl, C₅-C₁₀-Cycloalkyl oder C₆-C₁₄-Aryl stehen, wobei diese Alkyl-, Cycloalkyl- und Arylreste gegebenenfalls mit Hydroxy- und/oder NH₂-Gruppen substituiert sein können und
n für 1 oder 2 steht,
oder aromatische Amine der Formel (II) 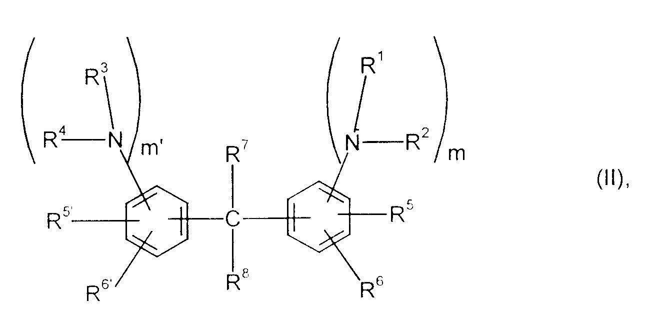 in der
R¹ bis R⁶ die bei Formel (I) angegebene Bedeutung haben,
R^{5'} und R^{6'} dem Umfang nach die bei Formel (I) für R⁵ und R⁶ angegebene Bedeutung haben, jedoch von R⁵ und R⁶ verschieden sein können,
R⁷ und R⁸ gleich oder verschieden sind und jeweils für Wasserstoff oder C₁-C₁₀-Alkyl stehen und
m und m' gleich oder verschieden sind und jeweils für Null, 1 oder 2 stehen, wobei
m + m' mindestens 2 ist
oder aromatische Amine der Formel (III) 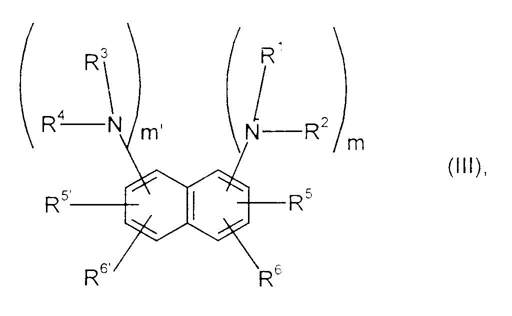 in der
R¹ bis R⁶ die bei Formel (I) angegebene Bedeutung haben,
R^{5'} und R^{6'} die bei Formel (II) angegebene Bedeutung haben und
m und m' die bei Formel (II) angegebene Bedeutung haben wobei
m + m' mindestens 2 ist

9. Verfahren nach Anspruch 7 oder 8, dadurch gekennzeichnet, daß man es bei 100 bis 360°C und 50 bis 400 bar durchführt und pro kg eingesetztes aromatisches Amin 0,05 bis 5 g Ruthenium (gerechnet als Metall) einsetzt.

## Claims

1. Catalysts, characterized in that they comprise ruthenium in metallic or combined form and carbonates, hydroxides and/or hydrated oxides of cerium and manganese and/or dehydration products thereof.

2. Catalysts according to Claim 1, characterized in that the ruthenium content is from 0.1 to 20 % by weight (calculated as elemental ruthenium), based on the total weight of the catalyst and the weight ratio of cerium to manganese (calculated as elements) is from 0 to 5:1.

3. Process for preparing ruthenium catalysts according to Claim 1, characterized in that soluble compounds of ruthenium, cerium and manganese, but not of chromium, are converted by treatment with alkaline compounds into insoluble compounds and these are optionally further treated, with the proviso that no Al₂O₃ support is present in this preparation of the ruthenium catalysts according to Claim 1.

4. Process according to Claim 3, characterized in that ruthenium is applied afterwards to the optionally further treated insoluble compounds of cerium and manganese or of manganese.

5. Process according to Claim 3, characterized in that the further treatment is carried out by filtration, washing free of soluble constituents, heat treatment, shaping and/or reduction of the ruthenium.

6. Process according to Claim 5, characterized in that a heat treatment is carried out, in which the material is dried at temperatures of up to 120°C and then calcined at temperatures of from 200 to 450°C.

7. Process for preparing cycloaliphatic polyamines by catalytic hydrogenation of corresponding aromatic amines in the presence of a catalyst, characterized in that the catalyst used comprises ruthenium in metallic or bonded form and carbonates, hydroxides and/or hydrated oxides of cerium and manganese and/or dehydration products thereof.

8. Process according to Claim 7, characterized in that the aromatic amines used have the formula (I) 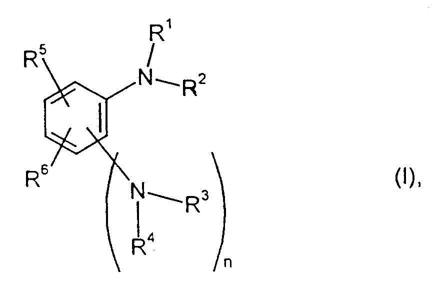 where
R¹, R², R³ and R⁴ are identical or different and in each case represent hydrogen or C₁-C₁₀-alkyl,
R⁵ and R⁶ are identical or different and in each case represent hydrogen, hydroxyl, C₁-C₁₀-alkyl, C₅-C₁₀-cycloalkyl or C₆-C₁₄-aryl, with these alkyl, cycloalkyl and aryl radicals optionally being able to be substituted by hydroxyl and/or NH₂ groups and
n represents 1 or 2,
or have the formula (II) 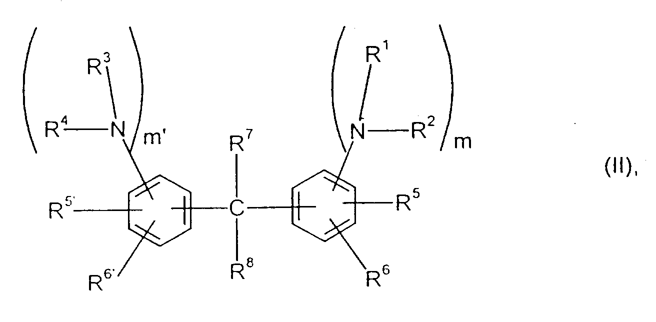 where
R¹ to R⁶ have the meanings given for formula (I),
R^{5'} and R^{6'} can, in terms of scope, have the meanings given for formula (I) for R⁵ and R⁶, but can be different from R⁵ and R⁶,
R⁷ and R⁸ are identical or different and in each case represent hydrogen or C₁-C₁₀-alkyl and
m and m' are identical or different and in each case represent zero, 1 or 2, with
m + m' being at least 2,
or have the formula (III) 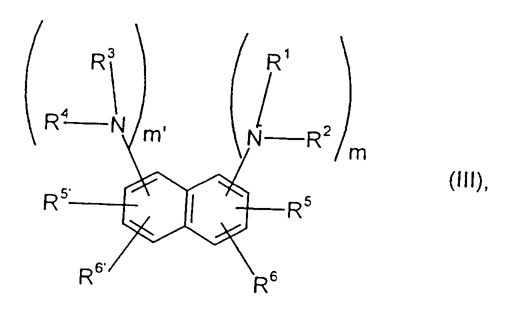 where
R¹ to R⁶ have the meanings given for formula (I),
R^{5'} and R^{6'} have the meanings given for formula (II) and
m and m' have the meanings given for formula (II), with
m + m' being at least 2.

9. Process according to Claim 8 or 9, characterized in that it is carried out at from 100 to 360°C and from 50 to 400 bar and from 0.05 to 5 g of ruthenium (calculated as metal) are used per kg of aromatic amine used.

## Revendications

1. Catalyseurs caractérisés en ce qu'ils sont constitués par du ruthénium sous forme métallique ou sous forme liée et par des carbonates, des hydroxydes et/ou des oxydhydrates du cérium et du manganèse et/ou de leurs produits de déshydratation.

2. Catalyseurs selon la revendication 1, caractérisés en ce que la teneur en ruthénium s'élève de 0,1 à 20% en poids rapportés au poids total du catalyseur (calculé comme ruthénium élémentaire) et le rapport pondéral du cérium au manganèse (calculés comme éléments) s'élève de 0 à 5:1.

3. Procédé pour la préparation de catalyseurs à base de ruthénium, caractérisé en ce qu'on transforme en composés insolubles des composés solubles du ruthénium, du cérium et du manganèse, mais non du chrome, par traitement avec des composés à réaction alcaline, et on soumet ces composés insolubles le cas échéant à un traitement ultérieur, avec cette mesure que, lors de cette préparation des catalyseurs à base de ruthénium selon la revendication 1, aucun support à base de Al₂O₃ n'est présent.

4. Procédé selon la revendication 3, caractérisé en ce qu'on applique par la suite du ruthénium sur les composés insolubles du cérium et du manganèse ou du manganèse soumis le cas échéant à un traitement ultérieur.

5. Procédé selon la revendication 3, caractérisé en ce que le traitement ultérieur a lieu par filtration, par élimination des constituants solubles par lavage, par traitement thermique, par façonnement et/ou par réduction du ruthénium.

6. Procédé selon la revendication 5, caractérisé en ce qu'on procède à un traitement thermique dans lequel on sèche à des températures allant jusqu'à 120°C, puis on calcine à des températures de 200 à 450°C.

7. Procédé pour la préparation de polyamines cycloaliphatiques par hydrogénation catalytique d'amines aromatiques correspondantes en présence d'un catalyseur, caractérisé en ce qu'on utilise un catalyseur qui est constitué par du ruthénium sous forme métallique ou sous forme liée et par des carbonates, par des hydroxydes et/ou par des oxydhydrates du cérium et du manganèse et/ou de leurs produits de déshydratation.

8. Procédé selon la revendication 7, caractérisé en ce qu'on met en oeuvre des amines aromatiques répondant à la formule (I) 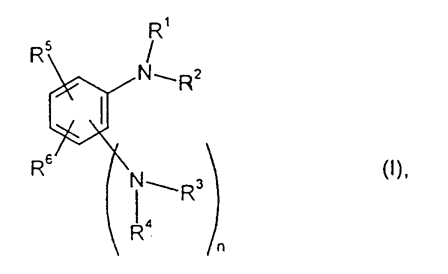 dans laquelle
R¹, R², R³ et R⁴ sont identiques ou différents et représentent respectivement un atome d'hydrogène ou un groupe alkyle en C₁-C₁₀,
R⁵ et R⁶ sont identiques ou différents et représentent respectivement un atome d'hydrogène, un groupe hydroxyle, un groupe alkyle en C₁-C₁₀, un groupe cycloalkyle en C₅-C₁₀ ou un groupe aryle en C₆-C₁₄, ces radicaux alkyle, cycloalkyle et aryle pouvant le cas échéant porter un ou plusieurs substituants hydroxyle et/ou NH₂, et
n représente 1 ou 2,
ou bien des amines aromatiques répondant à la formule (II) 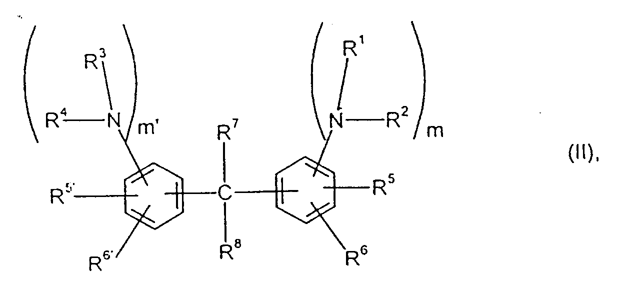 dans laquelle
R¹ à R⁶ ont la signification indiquée à la formule (I),
R^{5'} et R^{6'} ont la portée de signification indiquée pour R⁵ et R⁶ dans la formule (I); toutefois, ils peuvent être différents de R⁵ et de R⁶,
R⁷ et R⁸ sont identiques ou différents et représentent respectivement un atome d'hydrogène ou un groupe alkyle en C₁-C₁₀, et
m et m' sont identiques ou différents et représentent respectivement 0, 1 ou 2,
m + m' étant au moins égal à 2,
ou encore des amines aromatiques répondant à la formule (III) 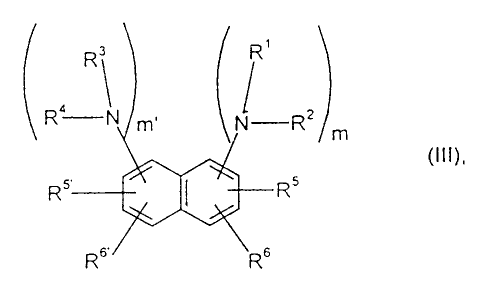 dans laquelle
R¹ à R⁶ ont la signification indiquée à la formule (I),
R^{5'} et R^{6'} ont la signification indiquée dans la formule (II), et
m et m' ont la signification indiquée dans la formule (II),
m + m' étant au moins égal à 2.

9. Procédé selon la revendication 7 ou 8, caractérisé en ce qu'on l'effectue à une température de 100 à 360°C et sous une pression de 50 à 400 bar, et on met en oeuvre par kg d'amines aromatiques mises en oeuvre, de 0,05 à 5 g de ruthénium (calculé comme métal).
